Europäisches Patentamt

(19) European Patent Office    (11) Publication number: **0 108 404**

Office européen des brevets    **B1**

(12)    **EUROPEAN PATENT SPECIFICATION**

(45) Date of publication of patent specification: **26.08.87**    (51) Int. Cl.⁴: **G 01 N 11/04**

(21) Application number: **83111033.3**

(22) Date of filing: **04.11.83**

(54) Process and equipment for determining or influencing the flow properties of solid granular materials.

(30) Priority: **04.11.82 HU 354582**

(43) Date of publication of application:
**16.05.84 Bulletin 84/20**

(45) Publication of the grant of the patent:
**26.08.87 Bulletin 87/35**

(84) Designated Contracting States:
**AT BE CH DE FR GB LI NL SE**

(56) References cited:
**DE-A-2 658 089**
**GB-A-1 165 313**
**US-A-4 320 657**

(73) Proprietor: **RICHTER GEDEON VEGYESZETI GYAR R.T.**
**Gyömröi ut 19-21**
**H-1475 Budapest X (HU)**

(72) Inventor: **Dávid, Agoston, Dr.**
**12, Batthyány u**
**H-1015 Budapest (HU)**
Inventor: **Pogány, János**
**140, Sasadi u**
**H-1112 Budapest (HU)**
Inventor: **Rönkös, László**
**47, Bodnár u**
**H-1163 Budapest (HU)**

(74) Representative: **Lehn, Werner, Dipl.-Ing. et al**
**Hoffmann, Eitle & Partner Patentanwälte**
**Arabellastrasse 4**
**D-8000 München 81 (DE)**

Courier Press, Leamington Spa, England.

**Description**

The invention relates to a process for determining flow properties of solid granular material, in a measuring cycle of which a sample to be tested is placed into a sample-holder means having changeable geometry and a controllable discharge aperture which can be controlled in accordance with either a mass-proportional or a time-proportional mode of operation, whereby

(a) in the mass-proportional mode of operation, passing of the sample through the discharge aperture is monitored and at the moment the sample-holder means becomes empty a signal representing the whole period of outflow is generated, and

(b) in the time-proportional mode of operation, after the expiration of a period prescribed in the measuring cycle a signal representing the mass of the outflowed sample is generated and connected to an input of the signal processing unit, thereafter by changing at least one parameter of the outflow of the material in a measuring sequence for each cycle, signals representing a value of at least one constant parameter and a value of a parameter changeable in each cycle are generated and supplied to a signal processing unit, which operates to supply a resultant signal in dependence thereon.

The invention also relates to apparatus for carrying out the process.

Such a process and such apparatus are known from DE—A—2658089.

The invention was developed for the determination of the flow-properties of finely-granular solid materials, powders and it will be described furtheron in relation to this field of application. However, the invention is not at all restricted to granular materials being finer than the defined limit value and for those, skilled in the art it will be quite clear that the invention is well suitable for examining coarser granular materials too, if a sample-holder can be prepared, in which materials may pass under the same conditions as in technological devices conventionally applied for passing materials with coarser grains.

In all professional fields interested in powder-technology, in particular in the pharmaceutical industry and others engaged in producing plant protecting agents, fodder, foodstuff, cosmetics, household-goods, etc., determination of flow properties of granular powders and influencing same by technological means is of utmost importance, in order to optimize transport, charging and compressing of powders and similar operations and processes, based on calculation, as well as to ensure proper quality of products.

Possibilities for advantageous solution have been frequently examined and several methods are known, such as from DE—A—26 58 089. However, neither a test-method, nor an equipment is known, which would enable determination of all parameters influencing flowing properties and a control of process (technological optimization) based on said properties. The known solutions are suitable for determination of only one part of the important characteristics together determining the process, and only this part can be considered for the controlling parameters. Accordingly, by using the devices corresponding to the state of art satisfactory optimization can be achieved only on the basis of empirical information.

The velocity of flow of certain powdery products discharged from a given tank has been examined thoroughly, but only in respect of some important parameters. R. L. Brown and J. C. Richards (Trans. Inst. Chem. Eng. *38*, 243 (1960) examined the role of the grain size of the powder and the diameter of the discharge aperture, also taking into consideration the density of the grains, but the correlation explored contained more constants of unknown nature, than the parameters considered as known. This correlation can be applied in practice only with severe restrictions.

Of the parameters influencing outflow velocity of powders, the effects of various parameters have been investigated:

— the effect of the height of the powder column and grain size (F. Q. Danish and E. L. Parrot, J. Pharm. Sci. *60*, 548, 1971);
— effect of moisture content (D. J. Craik and B. F. Miller, J. Pharm. Pharmac. *10*, 136T, 1958);
— effect of grain size distribution and the diameter of the aperture, (T. M. Jones and N. Pilpel, J. Pharm. Pharmac. *18*, 429, 1966);
— the minimal diameter required for outflow, the effect of grain size, grain density and geometry of grain shape, (K. Kurihara, I. Ichikawa, Chem. Pharm. Bull. *21*, 394, 1973).

All these investigations were performed with apparatus provided with a powder-tank with a discharge opening having changeable diameter. Generally, in this context change does not mean any arbitrary change of the aperture diameter within a fixed range, but rather switching over between the closed and open state, independently of whether closing and opening can be performed for one single value of the diameter or whether with the exchangeability of the structure element containing the discharge aperture according to a size order, the inserted outflow opening could optionally be closed or opened. Accordingly, hereinafter we shall use the expression "it can be opened and closed" instead of the attribute "changeable". In the known apparatus with the aid of the opening, the mass of the powder discharged in a longer interval was determined. In the course of measuring, the so-called "Flowometer" was used provided with an agitator, as well as an instrument suitable for measuring the shearing force. All these devices are suitable for determining only one group of the parameters to be considered and to define the empirical correlations between said preferential parameters.

From our earlier Hungarian Patent 174 116 a solution is known which ensures advantageous apparatus-technical conditions for the determination of flow properties of solid granular materials with the simultaneous observance of different characteristics; said solution enables the application of a process, comprising keeping two characteristics out of three always on a constant value and measuring the third parameter as an independent variable. The data obtained from the mass of facts by mathematical analysis on the place of examination, are suitable for the determination of the data serving as a starting point for the optimalization of operational processes in course of production or processing. From the aforementioned it becomes obvious that said solution enables but an analysis starting from variables of restricted number.

An object of our invention is to develop a measuring method, where the regularities of change of flowing properties are not to be approached empirically, but a quantitative correlation can be established between all important parameters influencing said properties of granular powders by one single mathematical formula.

Our previous invention (according to the cited Hungarian patent) was further developed. The modified apparatus was provided with further elements, and the mass of facts serving as the starting parameters of the analysis was determined by a new method, by the new apparatus and in compliance with it the mathematical correlation was elaborated, according to which data processing technique can be formed. By connecting the devices in accordance with said correlation, electrical signals can be produced directly from the mass of facts serving for the control of the process (revealing factual data and producing process controlling signals). The signals can be stored in memories known per se or forwarded to process control means.

As used in our previous solution, the sample was placed in sample-holder, preferably in the shape of a funnel, the discharge aperture of which could be opened and closed, and which could be controlled in two modes of operation (mass-proportional or time-proportional). Thereafter, by changing some parameters of the outflow of the medium (size of the aperture, duration etc.) in the single cycles, we produced—through the set of cycles—the signals representing the constant parameter(s) and the values of the parameters changing in the single cycles respectively. The signals were forwarded to the proper input(s) of the evaluating unit, while the result-signals produced by the evaluation unit were displayed and/or recorded and/or read.

According to a first aspect of the present invention, the process defined in the first paragraph of this specification is characterised in that prior to beginning each measuring cycle, preferably when filling the sample into the sample-holder means a signal representing the type of sample-holder means used in the current cycle and a signal representing the size of the discharge aperture used in said cycle are generated; said signals are supplied to inputs of the signal processing unit which also contains a function evaluator; thereafter the desired mode of operation is set and a parameter signal representing the constant parameter in the selected mode of operation is generated, said parameter signal is fed to an input of the signal processing unit, and the discharge aperture is opened, thereafter the signal processing unit operates at least one program to evaluate at least one function of the material outflow parameters.

According to a second aspect of the present invention, there is provided apparatus for carrying out the process of said first aspect and comprising:

sample-holder means having changeable geometry and a controllable discharge aperture;
means for controlling said discharge aperture;
means for receiving the sample;
means for generating signals representing a value of at least one constant parameter and a value of a parameter changeable in each cycle; and
a signal processing unit connected to receive said signals and to provide a resultant output signal; characterised by:
means for generating a signal representing the type of sample-holder means used in the current cycle;
means for generating a signal representing the size of the discharge aperture used in that cycle;
means for supplying said signals to said signal processing unit;
a function evaluator in said signal processing unit; and
means for causing said function evaluator to evaluate at least one function of the material outflow parameters.

In the process according to the invention, an exchangeable set of sample-holders with different geometry parameters (receiving equal sample quantities in different column heights) may be used, and prior to the start of each measuring cycle, the sample medium may be filled into the sample-holder, whereafter a signal representing the type of the sample-holder (geometrical characteristics) and a signal representing the size of the discharge aperture may be produced. Thereafter the signals are led to the proper inputs of the signal processing unit, if necessary the desired mode of operation is set and a signal representing the chosen mode of operation may also be supplied to the proper inputs of the signal processing unit. In case of a mass-proportional mode of operation a signal representing a preselected mass of the sample-medium is also supplied to the relating input of the signal processing unit.

In case of a time-proportional mode of operation, according to one version, a signal representing a preselected span of time is connected to the suitable input of the signal processing unit, while according to an improved version reference span of time is not pre-signalled to the signal processing unit, but in each measuring cycle the actual span of time—being close to the chosen span of time but slightly differing from time to time—is controlled and the signal representing it is led to the proper inputs of the signal processing unit.

Thereafter the discharge aperture is opened and depending on the mode of operation we proceed as follows:

— in the case of the mass-proportional mode of operation we follow the outflow of the sample from the sample-holder and in the moment when the sample-holder is empty the signal representing the full span of time of discharge is produced and led to the corresponding input of the signal processing unit.

— in case of time-proportional mode of operation the aperture is closed after the expiration of the span of time prescribed for the given measuring cycle, the signal representing the mass of the discharged sample-medium is produced and connected to the proper input of the signal processing unit, thereafter the signal processing unit is programmed according to the equation arranged for the dependent variable we are searching for, the signal appearing on the result output is displayed and/or recorded and/or read, and if desired the resulting signal, or the signal having been derived therefrom in a known manner, is connected to the control input(s) of the intervening device(s) modifying the parameter(s) of the technological process.

Signal processing is preferably controlled according to one of the correlations as described below:

The apparatus according to the invention can be preferably used for the realization of the process according to the invention. Taking into consideration the various means available in the field of modern measuring techniques, automation and in particular in the field of process control and electronic digital data processing, it goes without saying that the process can be realized by means of differently designed or coupled intervening devices. Measuring technical and process controlling systems of general destination may be used, if the field of application includes the fulfillment of the tasks of said type or devices developed for the process according to the invention, which can be inserted into known activating and signal processing systems serving simultaneously for several other processes at the corresponding place. Even when using such devices of more general purpose, the device system prepared for said purpose prior to performing the process will have the general characteristics described hereinafter.

The equipment according to the invention may be provided with a set of sample holders consisting of exchangeable sample holders realizing identical volumetric measures with different geometrical parameters (while the value of "cross-section×height" is constant the values of factors are varied) or it may be provided with one single sample-holder with a set of exchangeable sample holding inserts fitting thereto. Another possibility is a sample-holder with changeable geometry. A type signal-transmitter and an aperture signal-transmitter are preferably connected to the corresponding points of the sample holder, below the discharge aperture of the sample holder a mass signal-transmitter, preferably a balance, is preferably arranged, while the evaluating unit may be represented by an electronic signal processing unit, to the input(s) of which the type-signal transmitter, aperture-signal transmitter, period-signal transmitter, mass-signal transmitter and means for observing the path are connected.

The signal transmitters connected with the corresponding input(s) of the evaluating unit are elements which can be designed by a method known per se, and from the aforementioned the function thereof, becomes obvious, i.e. how the reference signal sources—formed in the usual manner—are performing the functions thereof by omitting the optional transmitters.

The output signal of the type-signal transmitter is the type-signal of the sample-holder (insert) out of the exchangeable sample-holders (inserts) used in the given measuring phase, delivering the information for the signal processing unit about the column-height and the volumetric measure to be realized thereby.

The aperture-signal transmitter gives an output signal representing the useful cross-section of the discharge aperture.

The period-signal transmitter gives a signal representing the instant (open, closed) position of the aperture-closing organ, the constitutional changes of which deliver the start and stop signals, respectively, for performing time measuring in the time-signal transmitter or in the signal processing unit.

The device provided with suitably selected detectors (optical, capacitive etc.) for observing the path, observes the advance of the material in the discharge channel i.e. it delivers the state-changing phase-times needed for the derivation of the quantity to be measured (period of flow) for the mass-proportional mode of operation.

The mass-signal transmitter is preferably a balance, the output signal of which represents the mass of the sample medium flowed during the reference period chosen in the time-proportional mode of operation and accumulated on the balance. Even in case of a preselected reference period, said period can be transmitted to the signal processing unit in different ways. In certain measuring processes it may be sufficient to give the signal processing unit the nominal period as a selected parameter, in this case a period-signal transmitter set to a constant value is used, not to be coupled to some data-following signal input of the signal processing unit. However, if it is not intended to perform processing related to the nominal period, as so the desired accuracy can not be obtained (this matter will be discussed later in detail), period will be delivered by the previously mentioned optional period-signal transmitter, which is directly observing the span of time of the open state. In case, if reference to the nominal value is not sufficient, effective value of the sample mass can be observed as well.

4

By way of example, embodiments of the invention will be explained in detail by reference to the drawing, wherein:

Figure 1 is a block-schematic of the embodiment of the equipment according to the invention—serving as an example—reduced to a size facilitating understanding; and

Figure 2 is a flow diagram of a preferred process according to the invention.

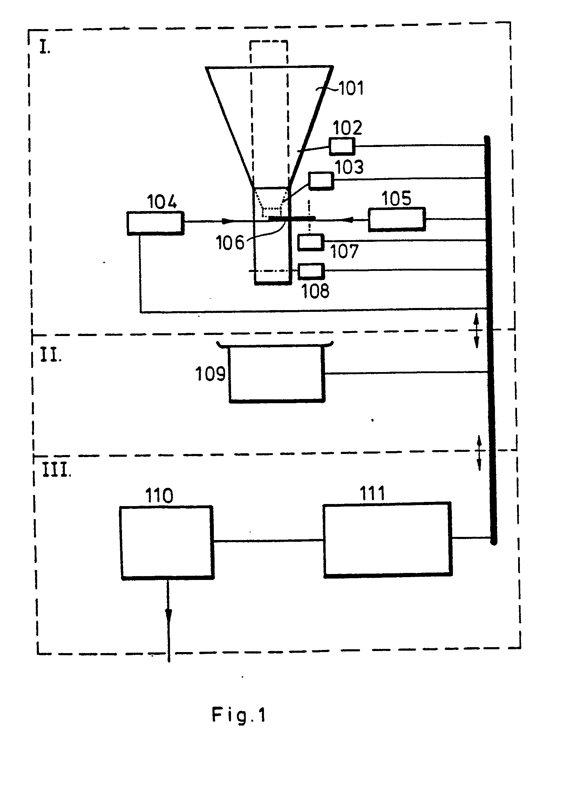

As can be seen in Figure 1, the set of devices forming the equipment can be divided into three main functional parts. The main part I. comprises the sample-holder 101 with the changeable geometry and the devices—designed in a well-known manner—partly observing, partly influencing the state thereof, namely the type-signal transmitter 102, the aperture-signal transmitter 103, the first executing element 104, the second executing element 105, the aperture-closing member 106, the period-signal transmitter 107 and the device 108 observing the path. The main part II. is formed by the mass-signal transmitter 109, generally a balance. The main part III. is formed by the signal processing unit 111 and the matching unit 110 connected thereto. With the embodiment serving as an example, a sample-holder—recommended also in the standards—is used, subtending an angle of 40°, with an aperture advantageously changeable in the range between 0.3 and 1.5 cm, which can be increased in height by using a cylindrical insert. The aperture-signal transmitter 103 signals the size of the aperture, while the type-signal transmitter 102 indicates the type of the sample-holder 101 used for the test. The device 108 observing the path observes the discharge aperture and indicates whether the sample medium is passing through or not.

The first and second executing elements 104, 105 provide for opening or closing of the aperture-closing member 106. The mass-signal transmitter 109 is preferably an electronic balance, delivering an output signal representing the weighed mass. The signals of the different signal transmitters and the signals representing the different reference values are processed in the signal processing unit 111, the resulting output—already mentioned in connection with the process—is represented by the matching stage 110 (Figure 1).

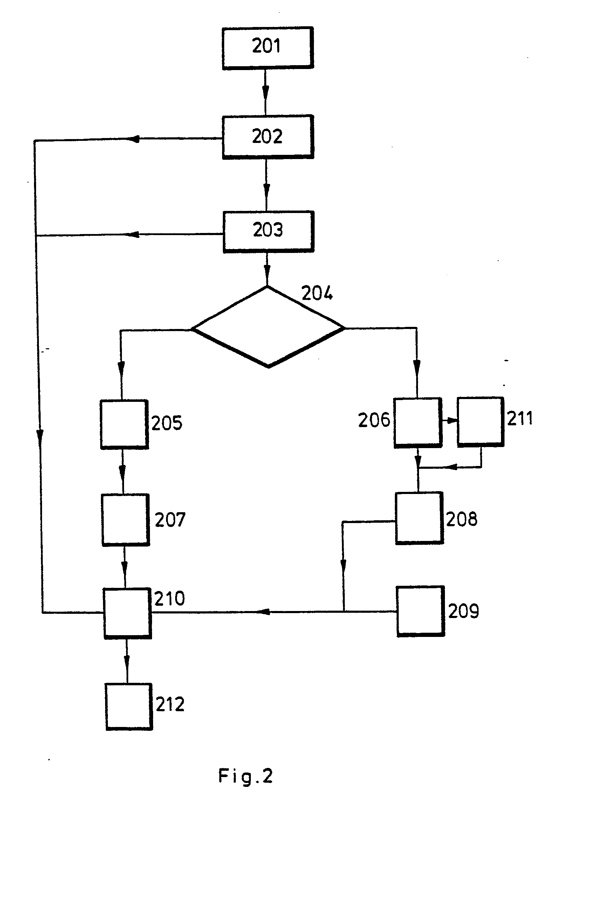

Operation of the equipment is illustrated by Figure 2. The sample of the material to be examined is put into the sample-holder 101. The corresponding signal transmitters forward data to the signal processing unit 111 concerning the type of the sample-holder used and the height of the sample column depending on the use of the inserts, as well as the size of discharge opening. Thereafter the mode of operation is selected. In case of a mass-proportional mode of operation the device 108 observing the path informs the signal processing unit 111 of the span of time, during which the nominal mass of the sample medium, has passed the discharge aperture. This can also be achieved as follows: the device 108 observing the path is a simple two-state structural element and by means of the signals representing the change in state in one or in the other direction as signals giving the starting and final phase, the signal processing unit 111 itself determines the duration of through-flow, so that the device 108 does not indicate the state change but delivers a signal representing the span of time passed between the two state changes. In case of time-proportional mode of operation the mass-signal transmitter 109 delivers the signal representing the mass passing through the time unit by a method known per se.

The signal processing unit 111 processes the data-signals received from the different signal-transmitters depending on the selected mode of operation and in accordance with the operation of a sequence of actions connected according to the algorithm ensuring the production of the searched quantity, thereafter it displays, records the result-signal(s) and forwards these or those derived from the result-signal to the corresponding input of the process controlling instrument.

The meanings of the steps illustrated in Figure 2:

201 starting the measuring process
202 signal generation indicating the type of the funnel and the height of the sample column,
203 signal generation indicating the size of the discharge aperture,
204 selection of the mode of operation,
205 mass-proportional mode of operation,
206 time-proportional mode of operation,
207 determination of the duration of the outflow in the mode 205 of operation,
208 determination of the duration of the open state in the mode 206 of operation,
209 weighing,
210 receipt of data, control, calculation,
211 actuating the aperture-closing member,
212 forwarding of the result signals (displays, recording, intervention).

The process and the equipment according to the invention enable the examination of solid granular materials, preferably by weighing the mass passing through the cross-section during a given time interval, expressed by the formula: $m/t[g \cdot s^{-1}]$; or by weighing the density of mass flow: the mass passing through unit cross section during the time unit, expressed by the formula: $m/t \cdot A[g \cdot s^{-1} \cdot cm^{-2}]$.

Duration of outflow of a sample of nominal mass can be measured in the mass-proportional mode of operation, while by using the intervening signals derived from the data-signals representing the result of measuring (or by manual intervention) system characteristics can be optimalized within wide limits, so e.g. quantities relating to mass and/or time by the aperture size, by the grain-size, grain-density or grain-size-distribution (apparent density of the grain aggregate), or by factors relating to the shape of the grains.

Time-proportional mode of operation can be advantageously used for testing the physical characteristics of solid granular press materials, granulate-compositions and/or the regulation, optimalization of the production of press materials. As a further advantageous field of application finishing technology in the pharmaceutical industry should be mentioned.

As already mentioned before, examination for the same purpose has been performed—according to the state of art—on samples, the important characteristics of which (grain-diameter, grain density, grain size-distribution etc.) differed to a slight extent only. As a consequence, the examination did not enable a simultaneous comprehensive mathematical analysis of the characteristic parameters. Field of application of the process and equipment according to the invention is confirmed by the results of the tests performed on the samples according to Table 1.

TABLE 1

| No. | Material | $d_s$ | $\rho_s$ | $\rho$ | $d_{omin}$ |
|---|---|---|---|---|---|
| 1. | Lactose pellet | 0.095 | 1.42 | 0.74 | 0.5 |
| 2. | Lactose pellet | 0.068 | 1.43 | 0.76 | 0.4 |
| 3. | Lactose pellet | 0.055 | 1.44 | 0.77 | 0.4 |
| 4. | Poppy | 0.1 | 1.16 | 0.61 | 0.4 |
| 5. | Mustard-seed | 0.196 | 1.7 | 0.76 | 0.7 |
| 6. | Glass pearl | 0.322 | 2.47 | 1.52 | 1.2 |
| 7. | Glass pearl | 0.409 | 2.47 | 1.43 | 1.2 |
| 8. | Lead shot | 0.162 | 11.4 | 6.33 | 0.6 |

In the table $d_s$ stands for the average diameter of the grain in cm, $\rho_s$ for the grain density weighed by using the pychometric method, expressed in $g \cdot cm^{-3}$, $\rho$ stands for the apparent density of the grain aggregate expressed in $g \cdot cm^{-3}$ and $d_{omin}$ refers to the minimal diameter of the aperture, if the aperture is smaller, then $d_{omin}$ mass flow can not be measured in cm.

Lactose pellets were produced in a plant equipment with starch and poly- /vinyl-pyrrolydone/ as additives. The granular fractions separated in a sieve fell into the range 1.0—0.9, 0.7—0.63 and 0.63—0.5 mm, while average grain diameter $d_s$ was determined on basis of mass-proportion. For performing examination in time-proportional mode of operation a material sample of the mass of 40 to 100 g is placed into the sample-holder 101, whereafter the mass of the outflowing sample-medium, flowing through discharge openings of various diameters ($d_o$=0.3 to 1.5 cm) in each cycle is weighed at ten different spans of time. The series were repeated three times, and the standard deviation (±5%) of 30 measurings characterized the accuracy of test method.

Mass-flow Q and density of mass-flow Q/A thus determined are summarized in Table 2.

TABLE 2

| No. of the sample | ±δ% | Q / Q/A | $d_o$ 1.2 | 1.1 | 1.0 | 0.9 | 0.8 | 0.7 | 0.6 | 0.5 | 0.4 |
|---|---|---|---|---|---|---|---|---|---|---|---|
| 1. | 4.1 | Q | 19.82 | 15.8 | 12.3 | 9.62 | 6.50 | 4.45 | 2.78 | 1.80 | — |
|    |     | Q/A | 18.7 | 17.04 | 16.76 | 15.27 | 13.8 | 12.5 | 10.6 | 10.06 | — |
| 2. | 1.2 | Q | 20.97 | 16.8 | 13.2 | 10.17 | 6.75 | 4.70 | 3.12 | 1.96 | 0.80 |
|    |     | Q/A | 19.78 | 18.98 | 17.98 | 16.87 | 14.33 | 13.17 | 11.86 | 10.95 | 7.08 |
| 3. | 2.4 | Q | 21.15 | 17.15 | 13.3 | 10.57 | 6.86 | 4.75 | 3.19 | 1.97 | 0.85 |
|    |     | Q/A | 29.95 | 19.38 | 18.12 | 17.53 | 11.56 | 13.9 | 12.13 | 11.05 | 7.52 |
| 4. | 3.6 | Q | 16.33 | 13.3 | 10.4 | 7.83 | 5.67 | 3.59 | 2.28 | 1.42 | 0.64 |
|    |     | Q/A | 15.41 | 15.03 | 14.17 | 12.99 | 12.04 | 10.06 | 8.67 | 7.93 | 5.66 |
| 5. | 2.1 | Q | 17.45 | 13.55 | 10.1 | 7.12 | 5.30 | 3.30 | — | — | — |
|    |     | Q/A | 16.46 | 15.31 | 13.76 | 11.81 | 11.25 | 9.20 | — | — | — |
| 6. | 4.5 | Q | 25.55 | 18.23 | — | — | — | — | — | — | — |
|    |     | Q/A | 22.61 | 20.6 | — | — | — | — | — | — | — |
| 7. | 3.8 | Q | 24.75 | 18.49 | — | — | — | — | — | — | — |
|    |     | Q/A | 21.9 | 20.9 | — | — | — | — | — | — | — |
| 8. | 3.2 | Q | 212.9 | 162.0 | 121.0 | 86.32 | 66.59 | 44.39 | 28.24 | 20.08 | — |
|    |     | Q/A | 200.8 | 183.1 | 164.9 | 143.2 | 141.4 | 124.3 | 107.3 | 112.2 | — |

Analysing the data thus obtained we found that by the values of mass-flow $Q$ and density of mass-flow $Q/A$ new function-correlations can be set up, and some of the demanded characteristics can be derived from said quantities. So e.g. the correlation between $Q$ mass-flow, the average size diameter $d_s$ and aperture-diameter $d_o$ can be expressed by the following n-th degree expression:

$$Q_m = \frac{m}{t} = k_1 \frac{\rho_s}{d_s}[d_o - D_m]^n \times [d_o^n - k_2 d_o + k_3] \qquad (1)$$

wherein: $k$; $k_2$; and $k_3$ are constants characterizing material quality. $D_m$ restricts the ratio $d_o$ and $d_s$: a useful practical date: $D_m \geqq 3 \ d_s$; $D_m$=minimal aperture-diameter.

At the same time density of mass-flow $Q/A$ can be correlated to the parameter-spectrum determining flow behaviour of solid granular powders according to the following general expression:

$$Q/A = f(d_s, \ d_o, \ \rho_s, \ \rho, \ g, \ j, \ G) \qquad (2)$$

wherein the definition of the new symbols is as follows: g=gravitational acceleration factor, j=factor of rolling-sliding resistance, G=a constant characterizing material quality and simultaneously representing a geometric factor.

An example for the optimalization of the technology of a granulatum to be pressed to tablets can be found hereinafter.

According to an empirical solution of the function (2)

$$Q/A = \rho_s \cdot j \cdot \left( \frac{d_o^2}{d_s} \right) + G \cdot g \cdot \left( 1 - \frac{\rho_s^{-\rho}}{\rho_s \cdot \rho} \right) \qquad (3)$$

By the aid of the function (3) constants $j$ and $G$ can be derived from the measured data and taking these into consideration optimal parameters of the granulate can be calculated within reasonable limits.

Relative comparison is demonstrated by the test results data of the samples of Table 1.

TABLE 3

| Sample | $r^2$ | j | G |
|---|---|---|---|
| Lactose pellet 0.095 | 1.00 | 0.56 | 0.838 |
| Lactose pellet 0.068 | 0.98 | 0.42 | 0.790 |
| Lactose pellet 0.055 | 0.97 | 0.35 | 0.768 |
| Poppy | 0.97 | 0.66 | 1.147 |
| Mustard-seed | 0.98 | 0.87 | 0.910 |
| Lead shot | 0.98 | 1.12 | 1.016 |

In the above table $r^2$ stands for a regression coefficient. The data show that the coefficient j increases mainly by increasing grain density (in particular in case of lead-shot), unevenness of the grain surface (in particular in case of poppy and mustard-seed), i.e. the rolling-sliding resistance increases, the constant G is increased by the apparent density of the grain aggregate (including grain-size-distribution), as well as by grain density (in particular in case of lead-shot, but also in case of mustard-seed). Geometry of poppy is ellipsoid and not spherical-symmetrical, the value of the constant G is sensitive also to said geometry.

Accordingly, a granulate-composition to be optimized is prepared with different composition, moisture content and eventually by using various technological processes. Thereafter the mass-flow $Q$ and density of mass-flow $Q/A$ values of the experimental sample-series are measured according to the invention.

For the tablets to be produced of diameter $d_o$ and of mass m—by using the formula $m = Q \cdot t$ the time t, can be obtained which is needed for filling the die. The best tabletting equipment can be chosen accordingly.

If no granulate with suitable mass-flow can be found in the series of samples, then by the aid of the density of mass-flow values $Q/A$ calculated for the different aperture-diameters, on basis of the formula (3) the parameters $d_o$, $d_s$, $\rho_s$ and $\rho$ adapted to the optimally lowest constants j and G can be determined.

8

The optimal parameters are then ensured in the course of granulate production by using the known technological methods—not empirically.

As already mentioned before, the time-signal transmitter is not necessarily the time-signal transmitter 107 sensing directly actual span of time of the open state. If measuring accuracy is satisfactory by considering the selected nominal span of time, the time-signal transmitter might be a signal source set to the nominal value, so the equipment will be provided only with such a device, (signal source may even form a part of the signal processing unit, either in form of software, or hardware). It may happen that the period-signal transmitter 107 which can be seen in Figure 1, is able to ensure desired accuracy with a corrector only. The period-signal transmitter 107 has a position-signal transmitter being sensitive to the two final positions of the aperture-closing member 106, which—after having reached one or the other final position—is giving an output pulse representing the change of state (change of state in direction of closing or opening). If the signal processing unit 111 is used for generating the signal representing the span of time between the points of time of the two changes in state, the period-signal transmitter 107 consists only of the position-signal transmitter delivering the two state-signals needed for the derivation of the span of time. In another embodiment the period-signal transmitter 107 is designed so, as to give an output signal representing directly the span of time.

However, in both embodiments this type of signal formation will give an accurate value only, if the transients of the displacement between the two extreme positions of the aperture-closing member 106 are the same in both senses (if through the gradually widening i.e. narrowing aperture the medium is allowed to pass). If the specific transmission values of the transient in the closing respectively, in the opening direction are not equivalent values, transient process is to be followed with the sensors. This can be performed so, that the period-signal transmitter 107 consists only of the densors observing the transient and the signal processing unit 111 generates the period-signal taking the signals of the two extreme positions and the transient signal as a basis or on basis of the two state-signals and the transient signal the time-signal transmitter 107 is generating the signal representing directly the periods, which is then led into the signal processing unit 111.

Changing of the geometry of the sample-holder 101 can be realized in several ways. From the point of view of construction it seems to be the most simple solution to prepare a set of sample holders with different column heights and discharge apertures, these can be inserted into a suitable place within the equipment. The changeable set of elements, however, is of a large volume. A smaller volume can be obtained, if in one common funnel insert elements modifying the geometry are placed.

In further preferred embodiment below the lower aperture of the funnel a disc is rotated in a stepping mode of operation which is formed with a set of permeable apertures with different cross-sections. In this embodiment the sample-holder is formed as a funnel-shaped body, below the lower aperture of which a disc is arranged, which can be rotated excentrically to the central axis of the funnel and along the peripheral arc of the disc intersecting the central axis of the funnel permeable openings are formed with different cross-sections one after the other, while the element of the disc enabling the stepping motion is provided with a blocking device; the blocking positions occupying an angular position in which one of the apertures, allowing passage through the sample, covers the lower aperture of the funnel.

By giving some special embodiments of the invention the above examples were to illustrate that in order to realize the fundamental characteristics of the process only some fundamental characteristics have to be realized similarly, while structural design can be most diverse, the invention only being limited by the scope of the claims.

## Claims

1. Process for determining flow properties of solid granular material, in a measuring cycle of which a sample to be tested is placed into a sample-holder means (101) having changeable geometry and a controllable discharge aperture which can be controlled in accordance with either a mass-proportional or a time-proportional mode of operation, whereby

(a) in the mass-proportional mode of operation, passing of the sample through the discharge aperture is monitored and at the moment the sample-holder means becomes empty a signal representing the whole period of outflow is generated, and

(b) in the time-proportional mode of operation, after the expiration of a period prescribed in the measuring cycle a signal representing the mass of the outflowed sample is generated and connected to an input of the signal processing unit,

thereafter by changing at least one parameter of the outflow of the material in a measuring sequence for each cycle, signals representing a value of at least one constant parameter and a value of a parameter changeable in each cycle are generated and supplied to a signal processing unit (111), which operates to supply a resultant signal in dependence thereon,

characterised in that:

prior to beginning each measuring cycle, preferably when filling the sample into the sample-holder means (101), a signal representing the type of sample-holder means used in the current cycle and a signal representing the size of the discharge aperture used in said cycle are generated; said signals are supplied to inputs of the signal processing unit (111) which also contains a function evaluator; thereafter the desired mode of operation is set and a parameter signal representing the constant parameter in the selected mode of operation is generated, said parameter signal is fed to an input of the signal processing unit, and the discharge aperture is opened, thereafter the signal processing unit operates at least one program to evaluate at least one function of the material outflow parameters.

2. A process according to claim 1 wherein said resultant signals are displayed and/or stored and/or read.

3. A process according to claim 1 or 2 wherein in the time-proportional mode of operation a signal is supplied to the signal processing unit representing nominal duration of said period prescribed in said measuring cycle.

4. A process according to any one of claims 1 to 3 wherein in the mass-proportional mode of operation a signal is supplied to the signal processing unit representing a nominal mass of the sample.

5. A process as claimed in any one of claims 1 to 4 wherein in the time-proportional mode of operation the actual duration of the open state of the discharge aperture is monitored and a signal representing this duration is supplied to the signal processing unit.

6. A process as claimed in any one of claims 1 to 5 wherein the signal generated in the course of the current measuring cycle and representing nominal or actual mass of the sample and the period-signal representing the nominal or actual duration of the open state of the discharge aperture are supplied to the signal input of a quotient forming circuit of the signal processing unit and a quotient signal thus obtained is supplied to the signal input of said function-evaluator.

7. A process as claimed in claim 6 wherein said function evaluator is arranged to evaluate the n-th degree function

$$Q = \frac{m}{t} = k_1 \cdot \frac{\rho_s}{d_s} \cdot (d_o - D_m)^n \cdot (d_o^n - k_2 d_o + k_3) \tag{1}$$

in which $D_m$ stands for the minimal diameter of the aperture, $Q$ is the mass-flow rate; $k_1$, $k_2$ and $k_3$ are constants characterising material quality, $d_s$ indicates the average grain size; $D_m \geqslant 3d_s$; $d_o$=the size of the discharge aperture, representing for example the diameter-value thereof; and $\rho_s$ represents the density of the solid grain.

8. A process as claimed in claim 6, wherein said function evaluator is arranged to evaluate the function

$$Q/A = f(d_s, d_o, \rho_s, \rho, g, j, G) \tag{2}$$

preferably

$$Q/A = \rho_s \cdot j \cdot \left( \frac{d_o^2}{d_s} \right) + G \cdot g \cdot \left( 1 - \frac{\rho_s^{-\rho}}{\rho_s \cdot \rho} \right) \tag{3}$$

wherein $Q$ is the mass flow rate, $d_s$ is the average grain size, $d_o$ is the diameter of the discharge aperture, A is the area of the aperture,

g=the coefficient of gravitational acceleration,

j=coefficient of rolling-sliding resistance,

G=coefficient of material quality, simultaneously representing a geometric coefficient,

$\rho_s$=density of the solid grain tested, and

$\rho$=virtual density of the grain aggregate.

9. A method of controlling a process in which a control device is arranged to modify at least one parameter of the process in response to a signal supplied to a control input characterised in that the control input is connected to receive said resultant signal generated according to the process of any one of claims 1 to 8.

10. A method as claimed in claim 9, wherein said process is the operation of granulating equipment and said control device is arranged to control the period needed for filling a die of said equipment.

11. Apparatus for carrying out the process of claim 1 and comprising:

sample-holder means (101) having changeable geometry and a controllable discharge aperture;

means for controlling said discharge aperture;

means for receiving the sample;

means for generating signals representing a value of at least one constant parameter and a value of a parameter changeable in each cycle; and

a signal processing unit (111) connected to receive said signals and to provide a resultant output signal;

characterised by:

means (102) for generating a signal representing the type of sample-holder means (101) used in the current cycle;

means (103) for generating a signal representing the size of the discharge aperture used in that cycle;

means for supplying said signals to said signal processing unit (111);

a function evaluator in said signal processing unit (111); and

means for causing said function evaluator to evaluate at least one function of the material outflow parameters.

12. Apparatus according to claim 11 wherein for use in said mass-proportional mode of operation a period signal transmitter (109) is provided to supply to said processing unit (111) a signal representing the period of outflow.

13. Apparatus as claimed in claim 12 wherein the period-signal transmitter (107) is provided with a position-signal transmitter sensitive to the two extreme positions of an aperture-closing member (106) and having a pulse output, furtheron it is preferably provided with a time to at least one input of which an output of the position-signal transmitter is connected.

14. Apparatus according to claim 11, 12 or 13 wherein for use in said time-proportional mode of operation a mass-signal transmitter (109) is provided to supply to said processing unit (111) a signal representing the mass of the outflowed sample medium.

15. Apparatus according to any one of claims 11 to 14 wherein the sample-holder means (101) comprises a funnel-shaped body, and below the lower aperture thereof a disc is arranged, which can be excentrically rotated in relation to the central axis of the said body in a stepping mode of operation, a sequence of apertures of differing cross-sections being provided along the periphery of said disc on an arc intersecting said axis, and wherein a stepping element for moving the disc is provided with a blocking member and the blocking positions are angular positions in which one of the different apertures covers the lower aperture of said body.

**Patentansprüche**

1. Verfahren zur Bestimmung von Fließeigenschaften von festem Granulatmaterial, in deren Meßzyklus eine Probe des zu testenden Materials in einer Probebehältervorrichtung (10) angeordnet wird, der eine änderbare Geometrie und eine steuerbare Ausflußöffnung aufweist, die in Übereinstimmung mit entweder einem massenproportionalen oder einem zeitproportionalen Betriebsmodus gesteuert werden kann, wodurch:

(a) im massenproportionalen Betriebsmodus das Durchfließen des Probemittels durch die Ausflußöffnung überwacht wird und im Zeitpunkt des Leerens des Probenbehälters ein Signal erzeugt wird, das die gesamte Periode des Ausfließens darstellt, und

(b) im zeitproportionalen Betriebsmodus nach dem Verstreichen der Periode, die zuvor im Meßzyklus beschrieben wurde, ein Signal erzeugt wird, das die Masse des ausgeflossenen Probemittels kennzeichnet und einem Eingang der Signalverarbeitungseinheit zugeführt wird, wonach durch Ändern mindestens eines Parameters des Materialausflusses in einer Meßfolge für jeden Zyklus Signale erzeugt werden, die einen Wert von mindestens einem konstanten Parameter und einen Wert eines in jedem Zyklus änderbaren Parameters darstellen und zu einer Signalverarbeitungseinheit (111) übertragen werden, durch die in Abhängigkeit hiervon ein resultierendes Signal lieferbar ist,

dadurch gekennzeichnet, daß:

vor dem Beginn jedes Meßzyklus, vorzugsweise beim Füllen der Probe in die Probenbehältervorrichtung (101), ein Signal, das den Typ der im laufenden Zyklus verwendeten Probenbehältervorrichtung darstellt, und ein Signal, das die Größe der in dem Zyklus verwendeten Ausflußöffnung darstellt, erzeugt werden; daß die Signale den Eingängen der Signalverarbeitungseinheit (111) zugeführt werden, die auch einen Funktionsauswerter enthält; daß danach der gewünschte Betriebsmodus eingestellt wird und ein Parametersignal, das den konstanten Parameter in dem ausgewählten Betriebsmodus darstellt, erzeugt wird, daß das Parametersignal einem Eingang der Signalverarbeitungseinheit zugeleitet wird, daß die Ausflußöffnung geöffnet wird, wo die Signalverarbeitungseinheit nach mindestens einem Programm zum Auswerten mindestens einer Funktion der Materialausfluß-Parameter arbeitet.

2. Verfahren nach Anspruch 1, dadurch gekennzeichnet, daß die resultierenden Signale angezeigt und/oder gespeichert und/oder abgelesen werden.

3. Verfahren nach Anspruch 1 oder 2, dadurch gekennzeichnet, daß im zeitproportionalen Betriebsmodus ein Signal der Signalverarbeitungseinheit zugeführt wird, welches die nominelle Dauer der Periode, die im Meßzyklus beschrieben wurde, verkörpert.

4. Verfahren nach einem der Ansprüche 1 bis 3, dadurch gekennzeichnet, daß im massenproportionalen Betriebsmodus ein Signal der Signalverarbeitungseinheit zugeführt wird, das eine nominelle Masse der Probe darstellt.

5. Verfahren nach einem der Ansprüche 1 bis 4, dadurch gekennzeichnet, daß in dem zeitproportionalen Betriebsmodus die tatsächliche Zeitdauer des Offenzustands der Ausflußöffnung überwacht wird und ein Signal, das diese Zeitdauer verkörpert, der Signalverarbeitungseinheit zugeführt wird.

6. Verfahren nach einem der Ansprüche 1 bis 5, dadurch gekennzeichnet, daß das Signal, das im Verlauf des laufenden Meßzyklus erzeugt wird und die nominelle oder tatsächliche Masse der Probe darstellt, und das Periodensignal, das die nominelle oder tatsächliche Zeitdauer des Offenzustandes der Ausflußöffnung verkörpert, dem Signaleingang einer Quotientenbildungsschaltung der Signalverarbeitungseinheit zugeführt werden und ein auf diese Weise erhaltenes Quotientsignal zum Signaleingang des Funktionsauswerters zugeführt wird.

7. Verfahren nach Anspruch 6, dadurch gekennzeichnet, daß der Funktionsauswerter zum Auswerten der Funktion des n-ten Grades

$$Q = \frac{m}{t} = k_1 \cdot \frac{\rho_s}{d_s} \cdot (d_o - D_m)^n \cdot (d_o^n - k_2 d_o + k_3) \tag{1}$$

ausgebildet ist, in der $D_m$ den Minimaldurchmesser der Ausflußöffnung darstellt, wobei $Q$=Massendurchfluß; $k_1$, $k_2$ und $k_3$ die Materialqualität charakterisierende Konstanten, $d_s$=Durchschnittskorngröße; $D_m \geqslant 3d_s$; $d_o$=Größe der Ausflußöffnung, und $\rho_s$ der Dichte des festen Korns entspricht.

8. Verfahren nach Anspruch 6, dadurch gekennzeichnet, daß der Funktionsauswerter zum Auswerten der Funktion

$$Q/A = f(d_s, d_o, \rho_s, \rho, g, j, G) \tag{2}$$

vorzugsweise

$$Q/A = \rho_s \cdot j \cdot \left( \frac{d_o^2}{d_s} \right) + G \cdot g \cdot \left( 1 - \frac{\rho_s^{-\rho}}{\rho_s \cdot \rho} \right) \tag{3}$$

angeordnet ist, wobei $Q$=den Massendurchfluß, $d_s$ die Durchschnittskorngröße, $d_o$ den Durchmesser der Ausflußöffnung und $A$ den Querschnitt der Ausflußöffnung bedeuten, und wobei

$g$=der Koeffizient der Gravitationsbeschleunigung,

$j$=der Koeffizient des Roll-Gleit-Widerstandes,

$G$=der Koeffizient der Materialqualität, der gleichzeitig einen geometrischen Koeffizienten darstellt,

$\rho_s$=die Dichte des getesteten festen Kornes, und

$\rho$=die virtuelle Dichte des Kornaggregates.

9. Verfahren zum Steuern eines Prozesses, in dem eine Steuervorrichtung zum Modifizieren mindestens eines Parameters des Prozesses ansprechend auf ein einem Steuereingang zugeführtes Signal angeordnet ist, dadurch gekennzeichnet, daß der Steuereingang angeschlossen ist, um das resultierende Signal entsprechend dem Prozess nach einem der Ansprüche 1 bis 8 zu empfangen.

10. Verfahren nach Anspruch 9, dadurch gekennzeichnet, daß der Prozeß den Betrieb von Granuliergeräten beinhaltet und die Steuervorrichtung zum Steuern der zum Ausfüllen einer Form des Geräts benötigten Periode ausgebildet ist.

11. Gerät zur Durchführung des Verfahrens nach Anspruch 1 enthaltend:

eine Probenbehältervorrichtung (101) mit veränderbarer Geometrie und einer steuerbaren Ausflußöffnung;

eine Einrichtung zum Steuern der Ausflußöffnung;

eine Einrichtung zur Aufnahme der Probe;

eine Einrichtung zum Erzeugen von Signalen entsprechend einem Wert von wenigstens einem konstanten Parameter und einem Wert von einem in jedem Meßzyklus änderbaren Parameter; und

eine Signalverarbeitungseinheit (111), die zum Empfangen der Signale und zum Erzeugen eines resultierenden Ausgangssignals geschaltet ist,

gekennzeichnet durch:

eine Einrichtung (102) zum Erzeugen eines Signals entsprechend dem Typ einer Probenbehältervorrichtung (101), die im laufenden Zyklus verwendet wird;

eine Einrichtung (103) zum Erzeugen eines Signals entsprechend der Größe der im laufenden Zyklus verwendeten Ausflußöffnung;

eine Einrichtung zum Zuführen der Signale zur Signalverarbeitungseinheit (111);

eine Funktionsauswertungseinheit in der Signalverarbeitungseinheit (111); und

eine Einrichtung, durch die der Funktionsauswerter veranlaßt wird, wenigstens eine Funktion der Materialausflußparameter auszuwerten.

12. Gerät nach Anspruch 11, dadurch gekennzeichnet, daß ein Periodensignalübermittler (109) zur Verwendung im massenproportionalen Betriebsmodus zum Zuführen eines Signals entsprechend der Zeitdauer des Ausflusses der Verarbeitungseinheit (111) vorgesehen ist.

13. Gerät nach Anspruch 12, dadurch gekennzeichnet, daß der Periodensignalübermittler (107) mit einem Positionssignalübermittler versehen ist, der auf zwei extreme Positionen eines Öffnungs-Schließ-Gliedes (106) anspricht und einen Impulsausgang aufweist, und der außerdem mit einem Zeitmasser ausgerüstet ist, an dessen Eingang der Ausgang des Positionssignalübermittlers angeschlossen ist.

14. Gerät nach Anspruch 11, 12 oder 13, dadurch gekennzeichnet, daß ein Massensignalübermittler (109) zur Verwendung im zeitproportionalen Betriebsmodus zum Zuführen eines Signals entsprechend der Masse der ausgeflossenen Probenmittel der Verarbeitungseinheit (111) vorgesehen ist.

15. Gerät nach einem der Ansprüche 11 bis 14, dadurch gekennzeichnet, daß die Probenbehältervorrichtung (101) einen trichterförmigen Körper aufweist, daß unter seiner unteren Öffnung eine Scheibe angeordnet ist, die exzentrisch im Bezug auf die Mittelachse des Körpers in einem schrittweisen Betriebsmodus gedreht werden kann, daß eine Reihenfolge von Öffnungen mit unterschiedlichen Querschnitten längs der Peripherie der Scheibe auf einem die Achse schneidenden Bogen vorgesehen ist, und daß ein Schrittelement zum Bewegen der Scheibe mit einem Blockierglied vorgesehen ist und die Blockierungspositionen Winkelpositionen sind, in denen eine der unterschiedlichen Öffnungen die untere Öffnung des Körpers bedeckt.

## Revendications

1. Un processus pour déterminer les propriétés d'écoulement d'un matériau granulaire solide, dans un cycle de mesure duquel un échantillon à tester est placé dans un moyen porte-échantillon (101) ayant une géométrie modifiable et une ouverture de décharge commandable pouvant être commandée selon un mode de fonctionnement soit proportionnel à la masse soit proportionnel au temps, de sorte que

(a) dans le mode de fonctionnement proportionnel à la masse, la traversée de l'échantillon par l'ouverture de décharge soit surveillée et, au moment où le porte-échantillon devient vide, un signal représentant l'intervalle de temps complet d'écoulement est produit, et

(b) dans le mode de fonctionnement proportionnel au temps, après l'expiration d'un intervalle de temps prescrit par le cycle de mesure, un signal représentant la masse de l'échantillon s'étant écoulée est produit et appliqué à une entrée de l'unité de traitement du signal,

puis, en changeant au moins l'un des paramètres d'écoulement du matériau dans une séquence de mesure pour chaque cycle, des signaux représentant une valeur d'au moins un paramètre constant et une valeur d'un paramètre modifiable dans chaque cycle sont produits et appliqués à une unité de traitement du signal (111) qui opère de manière à produire un signal résultant dépendant de ceux-ci,

caractérisé en ce que:

avant le début de chaque cycle de mesure, de préférence au moment du remplissage de l'échantillon dans le moyen porte-échantillon (101), un signal représentant le type de moyen porte-échantillon utilisé dans le cycle en cours et un signal représentant la taille de l'ouverture de décharge utilisée dans ce cycle sont produits; ces signaux sont appliqués aux entrées de l'unité de traitement de signal (111) qui contient également un évaluateur de fonction; puis le mode de fonctionnement désiré est choisi et un signal de paramètre représentant le paramètre constant dans le mode de fonctionnement choisi est produit, ce signal de paramètre est appliqué à une entrée de l'unité de traitement du signal et l'ouverture de décharge est ouverte, puis l'unité de traitement du signal exécute au moins un programme d'évaluation d'au moins une fonction des paramètres d'écoulement du matériau.

2. Le processus de la revendication 1, dans lequel lesdits signaux résultants sont affichés et/ou mémorisés et/ou lus.

3. Le processus de l'une des revendications 1 ou 2, dans lequel, dans le mode de fonctionnement proportionnel au temps, un signal est appliqué à l'unité de traitement de signal, qui représente la durée nominale de l'intervalle de temps prescrit dans le cycle de mesure.

4. Le processus de l'une des revendications 1 à 3, dans lequel, dans le mode de fonctionnement proportionnel à la masse, un signal est appliqué à l'unité de traitement du signal, qui représente une masse nominale de l'échantillon.

5. Le processus de l'une des revendications 1 à 4, dans lequel, dans le mode de fonctionnement proportionnel au temps, la durée réelle de l'état ouvert de l'ouverture de décharge est surveillée et un signal représentant cette durée est appliquée à l'unité de traitement de signal.

6. Le processus de l'une des revendications 1 à 5, dans lequel le signal produit pendant le cycle de mesure en cours et représentant la masse nominale ou réelle de l'échantillon, ainsi que le signal d'intervalle de temps représentant la durée nominale ou réelle de l'état ouvert de l'ouverture de décharge, sont appliqués à l'entrée de signal d'un circuit de formation de quotient de l'unité de traitement de signal, et un signal de quotient ainsi obtenu est appliqué à l'entrée de signal dudit évaluateur de fonction.

7. Le processus de la revendication 6, dans lequel ledit evaluateur de fonction permet d'évaluer la fonction du $n^{ieme}$ degré:

$$Q_m = m/t = k_1 \cdot \rho_s/d_s \cdot (d_o - D_m)^n \cdot (d_o{}^n - k_2 \cdot d_o + k_3) \tag{1}$$

dans laquelle $D_m$ représente le diamètre minimal de l'ouverture, $Q$ est la vitesse de débit massique; $k_1$, $k_2$ et $k_3$ sont des constantes caractérisant la qualité du matériau, $d_s$ indique la taille moyenne du grain; $D_m \geq 3 \, d_s$; $d_o$ représente la taille de l'ouverture de décharge, par exemple la valeur du diamètre de celle-ci; et $\rho_s$ représente la densité du grain solide.

8. Le processus de la revendication 6, dans lequel ledit évaluateur de fonction permet d'évaluer la fonction

$$Q/A = f(d_s, \, d_o, \, \rho_s, \, \rho, \, g, \, j, \, G) \tag{2}$$

de préférence:

$$Q/A = p_s \cdot j \cdot (d_o{}^2/d_s) + G \cdot g \cdot (1 - \rho_s{}^{-p}/\rho_s \cdot \rho) \tag{3}$$

dans laquelle Q est la vitesse de débit massique, $d_s$ est la taille moyenne des grains, $d_o$ est le diamètre de l'ouverture de décharge, A est la surface de l'ouverture,

g est l'accélération de la pesanteur,

j est le coefficient de résistance au roulement/glissement,

G est le coefficient de qualité des matériaux, représentant également un coefficient géométrique,

$\rho_s$ est la densité du grain solide testé, et

$\rho$ est la densité virtuelle de l'agrégat de grain.

9. Un procédé de commande d'un processus dans lequel un appareil de commande permet de modifier au moins un paramètre du processus en réponse à un signal appliqué à une entrée de commande, caractérisé en ce que l'entrée de commande est reliée de manière à recevoir ledit signal résultant produit par le processus de l'une quelconque des revendications 1 à 8.

10. Le procédé de la revendication 9, dans lequel le processus consiste à faire fonctionner un équipement de granulation, et dans lequel ledit appareil de commande permet de commander l'intervalle de temps nécessaire pour remplir une matrice de cet équipement.

11. Un appareil pour la mise en oeuvre du processus de la revendication 1, comprenant:

—des moyens porte-échantillon (101) comprenant une géométrie modifiable et une ouverture de décharge commandable,

—des moyens pour commander cette ouverture de décharge,

—des moyens pour recevoir l'échantillon,

—des moyens pour produire des signaux représentant la valeur d'au moins un paramètre de constante et la valeur d'un paramètre modifiable à chaque cycle, et

—une unité de traitement du signal (111) reliée de manière à recevoir lesdits signaux et à produire un signal de sortie résultant,

caractérisé par:

—des moyens (102) pour produire un signal représentant le type des moyens porte-échantillon (101) utilisés dans le cycle en cours,

—des moyens (103) pour produire un signal représentant la taille de l'ouverture de décharge utilisée dans ce cycle,

—des moyens pour appliquer lesdits signaux à l'unité de traitement du signal (111),

—un évaluateur de fonction, dans l'unité de traitement de signal (111), et

—des moyens pour permettre à cet évaluateur de fonction d'évaluer au moins une fonction des paramètres d'écoulement du matériau.

12. L'appareil de la revendication 11, dans lequel, pour une utilisation en mode de fonctionnement proportionnel à la masse, il est prévu un émetteur de signal d'intervalle de temps (109) pour appliquer à l'unité de traitement de signal (111) un signal représentant l'intervalle de temps d'écoulement.

13. L'appareil de la revendication 12, dans lequel l'émetteur de signal d'intervalle de temps (107) est pourvu d'un émetteur de signal de position sensible aux deux positions extrêmes d'un élément de fermeture de l'ouverture (106) et comprenant une sortie d'impulsion, et il est de préférence pourvu en outre d'un temporisateur à au moins l'une des entrées duquel une sortie de l'émetteur de signal de position est reliée.

14. L'appareil de l'une des revendications 11, 12 ou 13, dans lequel, pour l'utilisation en mode de fonctionnement proportionnel au temps, il est prévu un émetteur de signal de masse (109) pour appliquer à l'unité de traitement de signal (111) un signal représentant la masse de substance de l'échantillon qui s'est écoulée.

15. L'appareil de l'une quelconque des revendications 11 à 14, dans lequel les moyens porte-échantillon (101) comprennent un corps en forme d'entonnoir, avec un disque placé en dessous de l'ouverture inférieure de celui-ci, ce disque pouvant être entraîné en rotation autour d'un axe excentré par rapport à l'axe central du corps dans un mode de fonctionnement pas à pas, une suite d'ouvertures de sections différentes étant prévue le long de la périphérie de ce disque sur un arc intersectant ledit axe du corps, et dans lequel il est prévu un élément d'entraînement pas à pas du disque pourvu d'un élément de blocage, les positions de blocage correspondant à des positions angulaires dans lesquelles l'une desdites ouvertures différentes recouvre l'ouverture inférieure dudit corps.

Fig.1

Fig.2